# EUROPEAN PATENT SPECIFICATION

(11) **EP 3 554 443 B1**
(45) Date of publication and mention of the grant of the patent: **02.08.2023**
(21) Application number: 17822938.1
(22) Date of filing: 12.12.2017
(51) Int. Cl.: A61F 13/532, A61F 13/84, A61F 13/15

(54) **PROCESS FOR MAKING AN ABSORBENT CORE WITH ADDITIONAL PARTICLE MATERIAL**
VERFAHREN ZUR HERSTELLUNG EINES SAUGFÄHIGEN KERNS MIT ZUSATZTEILCHENMATERIAL
PROCÉDÉ DE FABRICATION D'UNE PARTIE CENTRALE ABSORBANTE CONTENANT UN MATÉRIAU PARTICULAIRE SUPPLÉMENTAIRE

(30) Priority: 19.12.2016 US 201662436020 P
(43) Date of publication of application: 23.10.2019
(73) Proprietor: The Procter & Gamble Company, Cincinnati, OH 45202 (US)
(72) Inventor: VAN DER KLUGT, Walter, 53881 Euskirchen (DE); ARMSTRONG-OSTLE, Peter, 53881 Euskirchen (DE); CALDWELL, Stuart Andrew, Newcastle Upon Tyne NE12 9BZ (GB)
(74) Representative: P&G Patent Germany
(86) International application number: PCT/US2017/065743
(87) International publication number: WO 2018/118514

(56) References cited:
- EP-A1- 2 119 420
- WO-A1-01/15646
- WO-A1-01/39707
- DE-A1-102010 043 113
- US-A1- 2006 004 335
- US-A1- 2015 045 756

## Description

### FIELD

The present disclosure relates to a process for making an absorbent core comprising superabsorbent polymer particles and additional particle material.

### BACKGROUND

In absorbent garments nonwoven fabrics are commonly used as a core cover to enclose the absorbent core. When used as core cover, the nonwoven fabric should contain the absorbent material that commonly comprises superabsorbent polymer material (SAP) which is typically applied as a powder or as fine particle material. The core cover should be designed to contain this material in dry state prior to use and also in use when the absorbent material may be contacted with bodily fluids.

In recent years effort has been made to decrease the amount of cellulose fibers, such as fluff pulp, used for the so-called "airfelt" in absorbent cores. Decreasing the amount is desirable for reasons of comfort and appearance due to less bulk in the crotch region. Furthermore, absorbent garments with reduced airfelt content occupy less storage space on the shelf, because they are thinner in the dry state prior to use.

The airfelt in conventional absorbent cores partly helps to immobilize the superabsorbent polymer material (SAP) in the dry and wet state, as the SAP particles are entangled between the airfelt fibers. Therefore, when the content of airfelt is reduced, other SAP-immobilization techniques are employed. For example in EP 1 447 066 (Busam et al.) the SAP is adhered to a substrate layer by using thermoplastic adhesive.

However, such immobilization techniques often require production processes wherein the nonwoven core covers have to endure relatively high strains compared to processes for producing conventional cores, comprising a comparably high amount of airfelt.

Thus, many absorbent cores that contain a high percentage of SAP still tend to be more likely to show a loss of SAP. Particularly in articles featuring an apertured topsheet, SAP lost from the core may get outside of the article and, when swollen due to the exposure to bodily fluids, stick to the wearers skin (so-called "gel on skin"), which is undesirable.

Furthermore, the airfelt in conventional absorbent cores can also partly help to absorb undesirable odors that may occur in dry and wet articles. Thus it may be desired to place an additional particle material, such as odor absorbing compositions or other particles that have health or consumer benefits, inside an absorbent core that has reduced airfelt. EP2119420 discloses a process for depositing superabsorbent particles onto a nonwoven.

### SUMMARY

The present disclosure relates to a process for making an absorbent core having a nonwoven core cover, superabsorbent polymer material and an additional particle material in accordance with claim 1.

### BRIEF DESCRIPTION OF THE DRAWINGS

Figure 1 shows an absorbent garment including an absorbent core with a core cover configured according to embodiments of the present disclosure.
Figure 2 shows an absorbent core with a core cover configured according to embodiments of the present disclosure.
Figure 3 shows another absorbent core with a core cover configured according to embodiments of the present disclosure.
Figure 4 shows a further absorbent core with a core cover configured according to embodiments of the present disclosure.
Figure 5 shows an embodiment of the process for making an absorbent core according to embodiments of the present disclosure.
Figure 6 shows a portion of the process of the embodiment of Figure 5.
Figure 7A shows a printing roll for use in the process of the embodiment of Figure 5.
Figure 7B shows indents on the printing roll of the embodiment of Figure 7A.
Figure 8 shows a support roll for use in the process of the embodiment of Figure 5.

### DETAILED DESCRIPTION

### Definitions

As used herein, the following terms have the following meanings:
"Absorbent article" refers to articles that absorb and contain liquid. In one embodiment, the term "absorbent article" refers to articles that are placed against or in proximity to the body of the wearer to absorb and contain the various exudates discharged from the body. Absorbent articles include but are not limited to diapers, adult incontinent briefs, training pants, diaper holders and liners, sanitary napkins and the like.

"Absorbent garment" refers to an absorbent article that is intended to be worn by wearer about the lower torso to absorb and contain the various exudates discharged from the body. Typically, an absorbent garment according to the present disclosure is disposable, however an absorbent garment can also be configured to be reusable.

"Diaper" refers to an absorbent garment generally worn by infants (e.g. babies or toddlers) about the lower torso. Suitable diapers are disclosed in U.S. Patent 5,221,274 issued to Buell et al. on June 22, 1993; and U.S. Patent 5,554,145 issued to Roe et al. on September 10, 1996. As used herein the term "diaper" also comprises "pant-like diapers": A pant-like diaper refers to an absorbent garment having fixed sides and leg openings or to a side-fastenable absorbent garment. Suitable pant-like diapers are disclosed in, e.g., U.S. Patent No. 5,246,433, issued to Hasse, et al. on September 21, 1993.

"Disposable" refers to items that are intended to be discarded after a limited number of uses, frequently a single use (i.e., the original absorbent article as a whole is not intended to be laundered or reused as an absorbent article, although certain materials or portions of the absorbent article may be recycled, reused, or composted). For example, certain disposable absorbent articles may be temporarily restored to substantially full functionality through the use of removable/replaceable components but the article is nevertheless considered to be disposable because the entire article is intended to be discarded after a limited number of uses.

"Comprise," "comprising," and "comprises" is an open ended term that specifies the presence of what follows e.g. a component but does not preclude the presence of other features, elements, steps or components known in the art, or disclosed herein.

"Absorbent core" refers to the structure and/or material of an absorbent article that is intended to absorb and store exudates discharged from the body. Generally, the absorbent core comprises an absorbent material, such as a superabsorbent polymer.

"Core cover" refers to a fabric, such as a nonwoven fabric, which is intended to at least partly cover and/or enclose an absorbent material of an absorbent core.

"Nonwoven fabric" refers to a manufactured web of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin. They may be staple or continuous filaments or be formed in situ. The terms "nonwoven fabric" and "nonwoven web" are used interchangeably in the present disclosure. The basis weight of nonwoven fabrics is usually expressed in grams per square meter (g/m²) and can be determined according to European Disposables and Nonwovens Association (EDANA) method 40.3-90. Generally, nonwoven fabrics may comprise fibers made by nature (natural fibers), made by man (synthetic fibers), or combinations thereof. Example natural fibers include but are not limited to: animal fibers such as wool, silk, fur, and hair; vegetable fibers such as cellulose, cotton, flax, linen, and hemp; and certain naturally occurring mineral fibers.

As used herein "strain" refers to the first substantial elongation of a nonwoven fabric to a length exceeding its initial length, wherein the initial length is the length of a sample of the nonwoven fabric directly after its manufacture. However, nonwovens may experience some minor, generally unintended elongations (insubstantial elongations) after manufacture. For example, the nonwoven fabric is wound up on a roll by the supplier. Insubstantial elongations typically do not extend the nonwoven to more than its initial length plus 2% or even only 1% of its initial length.

Herein "hot melt adhesive" is used according to the definition given in "Adhesion and Adhesives Technology: An Introduction" by Alphonsus V. Pocius (Hanser publishers Munich, 1997). Therein a hot melt is defined as an adhesive applied as a melt and gaining strength upon solidification.

As used herein, "zeolites" and "zeolite particles" are crystalline aluminosilicates of group IA and group IIA elements such as Na, K, Mn, Ca and are chemically represented by the empirical formula : M₂/nO, Al₂O₃, ySiO₂, wH₂O, where y is 2 or greater, n is the cation valence, and w is the water content in the voids of the zeolite.

### Absorbent garments

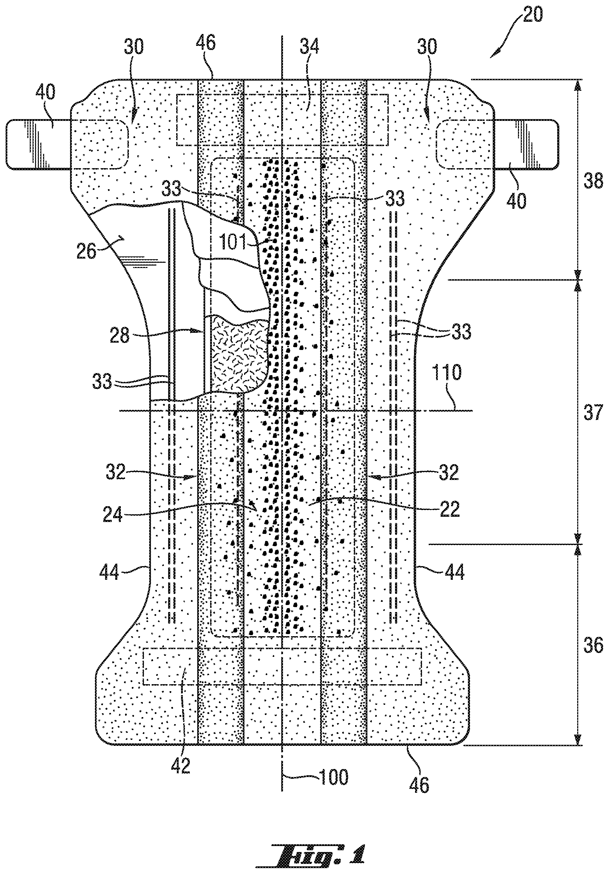

Figure 1 is a plan view of a diaper 20 as an embodiment of an absorbent garment according to the present disclosure. The diaper is shown in its flat out, uncontracted state (i.e., without elastic induced contraction). Portions of the structure are cut away to more clearly show the underlying structure of the diaper 20. The portion of the diaper 20 that contacts a wearer is facing the viewer. The chassis 22 of the diaper 20 in Figure 1 comprises the main body of the diaper 20. The chassis 22 comprises an outer covering including a liquid pervious topsheet 24 and/or a liquid impervious backsheet 26. The chassis 22 may also include most or all of the absorbent core 28 encased between the topsheet 24 and the backsheet 26. The chassis 22 may further include side panels 30, leg cuffs 32 with elastic members 33 and a waist feature 34. The leg cuffs 32 and the waist feature 34 typically comprise elastic members. One end portion of the diaper is configured as the front waist region 36 of the diaper 20. The opposite end portion is configured as the rear waist region 38 of the diaper 20. The intermediate portion of the diaper is configured as the crotch region 37, which extends longitudinally between the front and rear waist regions. The crotch region 37 is that portion of the diaper 20 which, when the diaper is worn, is generally positioned between the wearer's legs. The waist regions 36 and 38 may include a fastening system comprising fastening members 40 preferably attached to the rear waist region 38 and a landing zone 42 attached to the front waist region 36.

The diaper 20 has a longitudinal axis 100 and a transverse axis 110. The periphery of the diaper 20 is defined by the outer edges of the diaper 20 in which the longitudinal edges 44 run generally parallel to the longitudinal axis 100 of the diaper 20 and the end edges 46 run generally parallel to the transverse axis 110 of the diaper 20.

In one embodiment the topsheet of the absorbent garment of the present disclosure can also be apertured, i.e. the topsheet can have a plurality of apertures having an aperture size of at least about 0.2 mm². The topsheet may have an open area of at least about 10%, the open area being the sum of all apertures. The Method to determine the aperture size and open area of the apertured topsheet in context of the present disclosure is disclosed in EP 0953324.

In certain embodiments at least a part of the topsheet is apertured; for example apertured in at least 20%, or 50%, or 80%, or 90%, or 100% of the area overlaying the absorbent core. Due to the apertures the topsheet may not function as a second barrier for the SAP particles. Accordingly, embodiments of the present disclosure describe an absorbent core with improved SAP retaining properties for absorbent garments comprising an apertured topsheet.

A diaper may also include other features as are known in the art including front and rear ear panels, waist cap features, elastics and the like to provide better fit, containment and aesthetic characteristics.

An alternate configuration for absorbent articles is one for absorbent pants in which the central chassis structure does not extend to, or form, the front and rear waist edges of the pant. Rather, an elasticized belt structure entirely encircles the wearer's waist and forms the waist edge about the entire pant, and the side/hip panels. The central chassis is joined to the belt structure, usually on the inside thereof, with its ends disposed at locations in the front and rear waist regions somewhat below the waist edges of the belt structure. The elastic belt is usually relatively wide (in the longitudinal direction) and elastically stretchable in the lateral direction. It entirely encircles the wearer's waist, and thereby covers a relatively large amount of the wearer's skin. This configuration is sometimes known as a "belt" or "balloon" configuration (hereinafter, "belt" configuration).

In more detail, an absorbent article may have a front region, a rear region, and a crotch region disposed therebetween, further comprising a liquid permeable topsheet, a backsheet, and an absorbent core disposed between the topsheet and the backsheet. The article then may have a central chassis occupying the crotch region, and a belt structure disposed about the central chassis, the belt structure overlaying the backsheet to the outside thereof in the front and rear regions, and the belt structure overlapping and extending laterally and longitudinally outward from the chassis. The belt structure may comprise an outer nonwoven and an inner nonwoven and have elastic strands therebetween. The belt structure may further have a front belt portion having a front waist edge, and front left and right side edges; and a rear belt portion having a rear waist edge and rear left and right side edges, wherein the respective front and rear left side edges and the respective front and rear right side edges are joined, forming a waist opening and left and right leg openings.

Any pant configuration may have any of the article components described herein, for example, the topsheet, backsheet, core, or barrier cuffs described herein, along with an odor control composition, such as zeolites, and their placement. Further descriptions and embodiments of pant configurations may be found in U.S. Ser. No. 62/210635.

### Absorbent core

An absorbent core has two sides: an upper, body-facing side and a lower, garment-facing side. Furthermore an absorbent core comprises a core cover and absorbent material, comprising at least the SAP. According to the present disclosure the core cover, as described herein, may be used at least on one side of the absorbent core to cover at least a portion, or substantially all, or all of the respective side of the absorbent material.

Additionally, the core cover may also be used to cover at least a portion, or substantially all, or all of the body-facing side and the garment-facing side of the absorbent material, such that the absorbent material is wrapped by the core cover. In these embodiments the absorbent material may either be sandwiched between two separately provided sheets of core cover material, or wrapped by folding one sheet of core cover material, for example in a C-fold, to envelope the absorbent material.

When the nonwoven is intended to cover the body facing side of the absorbent core it may be desirable for the nonwoven to be hydrophilic. In certain embodiments of the present disclosure the nonwoven may be rendered hydrophilic by means known in the art.

In an alternative embodiment, the core cover may be used to cover only the garment-facing side of the absorbent material. However, in certain embodiments it may be preferred that the core cover described below covers the body-facing side of the absorbent material. In embodiments wherein a core cover comprises two separately provided sheets of material, at least one of the sheets can include a core cover material of the present disclosure.

In embodiments wherein a core cover comprises a single sheet of core cover material, the edges of the folded sheet may be sealed together to enclose the absorbent material. Sealing may be facilitated at least along the longitudinal edges of the absorbent core. Alternatively, the core cover may be sealed completely along all edges.

The amounts of materials used in the absorbent core herein are given in percent (%) by weight relative to the basis weight of the whole absorbent core. The whole absorbent core herein includes the core cover.

An absorbent core may comprise any absorbent material that is generally compressible, conformable, non-irritating to the wearer's skin, and capable of absorbing and retaining liquids such as urine and other certain body exudates. An absorbent core may comprise a wide variety of liquid-absorbent materials commonly used in disposable diapers or other absorbent articles. For example soft materials providing a rather fluffy structure with a lot of empty space, such as comminuted wood pulp, creped cellulose wadding, chemically stiffened, modified or cross-linked cellulosic fibers which are generally referred to as "airfelt". However, the absorbent core of the present disclosure preferably comprises less than 20%, or 15% or 10% or 5% by weight the absorbent core of such an airfelt material. The absorbent core may also be substantially free of, or completely free of airfelt material wherein "substantially free of" means that less than 1% by weight of the absorbent core is airfelt material and "completely free of" means that 0% by weight of the absorbent core is airfelt material.

The absorbent material comprises SAP, e.g. in the form of SAP particles, optionally mixed with fibrous materials. The absorbent core may comprise a relatively high amount of SAP of more than 80% or 85% or 90% or 95% by weight of the absorbent core. The absorbent core also comprises an additional particle material, such as zeolites. Furthermore, the absorbent core comprises a hot melt adhesive, as will be described in more detail below. According to one embodiment of the present disclosure the absorbent core comprises the superabsorbent polymer material, the additional particle material, the hot melt adhesive, and the core cover, wherein the amounts of these materials add up to present 99% or 100% by weight of the absorbent core.

An absorbent core according to the present disclosure may for example comprise a core cover a first nonwoven fabric and a second nonwoven fabric, wherein the SAP and additional particle material may be deposited on the first and second nonwoven fabrics respectively and hot melt adhesive may be deposited in such a way that it at least partly covers or enlaces the deposited SAP and additional particle material on the respective first and second nonwoven fabrics. The additional particle material, such as zeolites, may be deposited on either or both of the first and second nonwoven fabrics. In some embodiments, the zeolite particles may be deposited on only one of the nonwovens, either the nonwoven facing the topsheet or the nonwoven facing the backsheet. The absorbent core may then be incorporated in the absorbent garment in such that the first nonwoven fabric faces the topsheet. The first and optionally also the second nonwoven fabric may comprise the core cover of the present disclosure as will be described below.

It has now been found that production processes for absorbent cores comprising relatively high amounts of SAP of more than 80% or 85% or 90% or 95% by weight of the absorbent core and relatively low amounts of airfelt material of less than 20%, or 15% or 10% or 5% by weight of the absorbent core, and especially absorbent cores that are substantially free or even completely free of airfelt material, often involve steps where the core cover is exposed to higher strain compared to processes used for the production of conventional cores having comparably high amount of airfelt. These strains may cause damages, especially holes in the nonwoven fabric due to the rupture of fibers, and lead to an increased escape of SAP particles through these holes, or escape of other particles contained in the absorbent core, such as zeolites. Therefore the process of the present disclosure uses strain resistant nonwoven fabrics that can endure the strains occurring during such a process.

Further, an increased loss of SAP may occur when the article is in use. Due to uptake of fluids, the SAP swells, tends to expand, and may then be hydraulically forced through the core cover. This effect is even more pronounced in cores where the SAP particles are adhered to the core cover by hot melt adhesive, especially if the SAP particles are encapsulated or enlaced by the hot melt adhesive. Due to this encapsulation, the SAP particles may expand by tearing a hole through the core cover, since the expansion in other directions (away from the core cover) is hindered by the hot melt adhesive. This loss may cause the superabsorbent material to stick to a wearer's skin, a phenomenon commonly referred to as "gel on skin".

In view of the above-mentioned reasons core covers should be able to provide sufficient strength and integrity to survive strain intense production processes without substantial damage resulting in holes in the nonwoven and in loss of SAP or other particles such as zeolites through these holes. Therefore, a core cover of the present disclosure should retain the relatively small zeolite particles and SAP particles of the superabsorbent polymer material and simultaneously provide a strain resistant fabric that can be effectively employed in fast, strain intense production processes. Furthermore the core cover according to the present disclosure should withstand the exposure to strain when the absorbent article is in use, for example due to swelling of the superabsorbent polymer material.

### Process for making an absorbent core

The present disclosure refers to a process for making an absorbent core that comprises at least a first nonwoven core cover, a superabsorbent polymer material (SAP), and an additional particle material. The process comprises steps of (a) providing the first nonwoven core cover, (b) providing the SAP and additional particle material, (c) depositing the first nonwoven core cover on a support and (d) depositing the SAP and additional particle material on the first nonwoven core cover.

During the process, the first nonwoven core cover is strained. Typically, the strain occurring during such a process strains (elongates) the nonwoven by 10 - 20%, or 12 - 17%, or 12 - 14% of its initial length.

This strain is the first substantial strain the web undergoes after its manufacture. The strain may occur at any time during the production process and may be caused for example by the forces that are applied to hold the core cover on a support with an uneven or apertured surface. Such forces may for example be applied by vacuum means, pulling means (mechanically) or the like. The strain may as well be caused by laying down the SAP or other particles such as zeolites at high speed on the first nonwoven core cover. From the further description of the process of the present disclosure, it is apparent that in certain process executions the nonwoven core cover may locally experience slightly differing strains (for example in embodiments where the nonwoven is strained over a grid). Typically such locally differing strains are applied in a rather regular pattern and thus, for such embodiments, the process strains mentioned above will correspond to an average over the strained area.

It has been found that nonwoven core covers suitable for processes according to the present disclosure can be characterized by showing an increase in air permeability of less than 18% after having been strained by ε = 10% (wherein ε is prescribed strain, per Test Methods), or less than 20% after having been strained by ε = 15%. Suitable nonwoven core covers are described in more detail in the section "core cover".

The process may further comprise one or more steps of depositing a hot melt adhesive. The hot melt adhesive may be deposited in the form of fibers such that it enlaces and at least partly immobilizes the SAP and additional particles. In embodiments where the hot melt adhesive is applied in several steps, in each step a certain portion of the hot melt adhesive is applied. The overall amount of hot melt adhesive given in the section "hot melt adhesive" then corresponds to the sum of all portions of hot melt adhesive applied.

Additionally, the process may comprise steps of providing and depositing a second nonwoven core cover to cover the SAP and additional particle material. The second nonwoven core cover may be deposited such that the SAP, additional particle material, and the optional hot melt adhesive are sandwiched between the first and the second nonwoven core cover.

Alternatively, the process may comprise a step wherein the first nonwoven core cover is folded to wrap the SAP, additional particle material, and the optional hot melt adhesive. The first nonwoven core cover may be folded such that the SAP, additional particle material, and the optional hot melt adhesive are enveloped by the first nonwoven.

The steps of the process of the present disclosure will now be described in more detail.

### (a) Providing the first nonwoven core cover

The nonwoven may be taken from a roll where it is wound up, or it may be used directly after its manufacture without intermediate storage.

### (b) Providing the SAP

The SAP may be taken up from a reservoir, for example by a transfer device such as a hopper. The transfer device, such as a print roll, may have a radial outer surface and a rotational axis, with a plurality of recesses or cavities on the radial outer surface that can for example determine the amount and distribution pattern of SAP taken up by the transfer device. The SAP rotating the print roll about the rotational axis

### (c) Providing the additional or different particle material, such as zeolites.

The additional or different particle material may be provided by means of a suitable metering means, such as a screw feeder, vibratory tray, bulk solids pump, or the like, most preferably coupled with a loss in weight system for accurate dosing. The additional particles may also be taken up from a reservoir such as a hopper. The additional particle material may be in the same hopper as the SAP. The additional particle material may be fed into the hopper via a delivery pipe that penetrates the SAP bed in such a way that a cavity in the SAP bed is created into which the additional particle material may flow. The additional particle material may be from about 0.05 g to about 0.15 g per article, in some embodiments from about 0.01 g to about 0.2 g per article, and may be less than about 1% the content or weight of the absorbent core.

### (c) Depositing the first nonwoven core cover on a support

The support may possess an uneven or apertured surface. To provide an uneven surface, the support may comprise a plurality of cavities, indents, or grooves. A suitable support for example may be a support grid. The support has a first and an opposing second surface. Material, such as the first nonwoven core cover, will be deposited on the first surface. The deposited material may be held on the support by a drawing force, for example by gravitation, an air-stream or by a vacuum which can be applied on the second surface of the support. Thereby, any deposited material, such as the nonwoven core cover and/or the SAP and/or the additional particle material, will be held on the support by suction. Any apertured support on which deposited material can be held by means of passing air through the support herein may also be referred to as vented support.

The support may have the form of a plate, a grid or a belt, for example a rotating drum, a roll or a transport belt. In embodiments where the support is a drum, the first surface of the support corresponds to the outside surface of the drum and the second surface corresponds to the inside surface of the drum.

Due to the uneven or apertured surface of the support and the drawing force, the nonwoven core cover may be forced into an uneven shape; it may for example bulge corresponding to the apertures, indents or grooves.

### (d) Depositing the SAP and the additional particle material on the first nonwoven core cover

The SAP and additional particle material may be moved by the transfer device, such as a print roll, from the hopper to the first nonwoven core cover where the SAP and additional particle material may be rapidly deposited on the first nonwoven web. In some embodiments, the additional particle material's mass flow may be restricted to stay below the maximum conveying capability of the print roll. The SAP may be deposited on the nonwoven in such an amount that the content or weight of SAP in the finished absorbent core exceeds 80%, or 85%, or 90%, or 95% by weight of the absorbent core.

A process for forming an absorbent core or component of an absorbent article, may comprise the following steps:
providing a print roll having a radial outer surface and a rotational axis, wherein a plurality of cavities are defined in the radial outer surface;
rotating the print roll about the rotational axis;
providing a superabsorbent polymer particle material at a first input zone on the print roll;
providing a different particle material at a second input zone on the print roll;
partially filling at least some of the cavities with the different particle material at the first input zone during the rotation of the print roll;
partially filling at least some of the cavities with the superabsorbent polymer particle material at the second input zone during the rotation of the print roll;
bringing a substrate into a position proximate to or in contact with the print roll in a depositing zone;
depositing the superabsorbent polymer particle material on portions of the substrate in the depositing zone;
depositing the different particle material on portions of the substrate in the depositing zone; and
applying a hotmelt adhesive to the deposited superabsorbent polymer particle material and the deposited different particle material.

According to the present invention a process for forming an absorbent core or component of an absorbent article, comprises the following steps:
providing a print roll having a radial outer surface and a rotational axis, wherein a plurality of cavities are defined in the radial outer surface;
rotating the print roll about the rotational axis;
providing a superabsorbent polymer particle material at a second input zone on the print roll;
providing a different particle material at a first input zone on the print roll, wherein the different particulate material is an odor control composition comprising zeolite and/or silicas;
partially filling at least some of the cavities with the different particle material at the first input zone during the rotation of the print roll;
partially filling at least some of the cavities with the superabsorbent polymer particle material at the second input zone during the rotation of the print roll;
holding the superabsorbent polymer particle material and the different particular material in the at least some cavities between the input zones and the depositing zone using vacuum;
bringing a substrate into a position proximate to or in contact with the print roll in a depositing zone;
depositing the superabsorbent polymer particle material on portions of the substrate in the depositing zone;
depositing the different particle material on portions of the substrate in the depositing zone, wherein at least 70% of the different particle material is placed in a stripe substantially along a longitudinal axis of the substrate; and
applying a hotmelt adhesive to the deposited superabsorbent polymer particle material and the deposited different particle material.

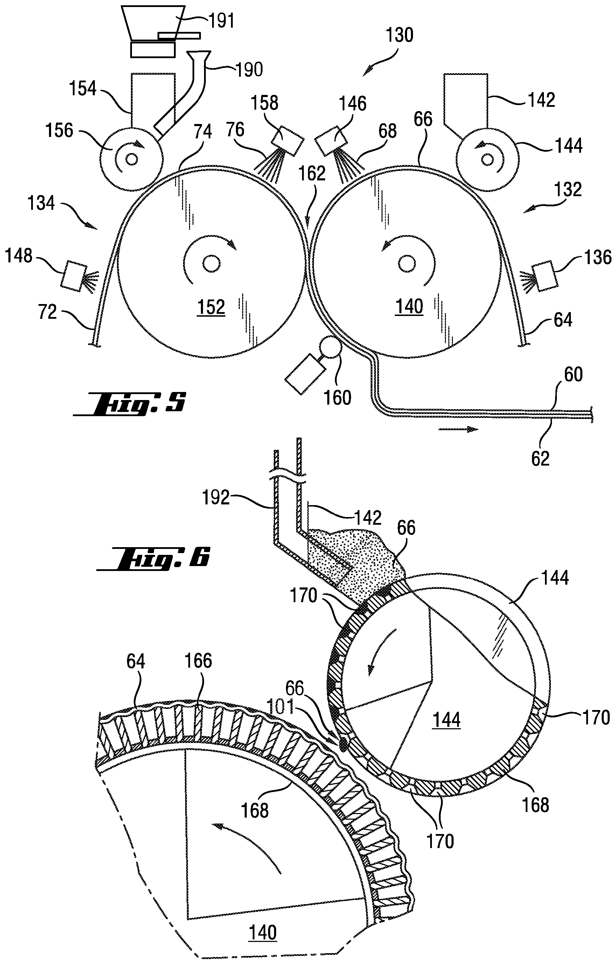

An example for a process of making an absorbent core according to the present disclosure will be described below with reference to Figures 5 - 8.

Printing system 130 for making an absorbent core 28 in accordance with certain embodiments of this disclosure is illustrated in Fig. 5 and may generally comprise a first printing unit 132 and a second printing unit 134 for forming the absorbent core 28.

In one embodiment the first printing unit 132 may comprise a first hot melt adhesive applicator (optional) 136 for applying a first portion of hot melt adhesive to the first nonwoven core cover 64, a first rotatable support roll 140 for receiving the first nonwoven core cover 64, a hopper 142 for holding SAP 66, an adhesive printing roll 144 for transferring the SAP 66 to the first nonwoven core cover 64, and a second hot melt adhesive applicator 146 for applying the hot melt adhesive (or a second portion of hot melt adhesive) 68 to the first nonwoven core cover 64 and the SAP 66 material thereon.

The second printing unit 134 may comprise a first hot melt adhesive applicator (optional) 148 for applying a first portion of hot melt adhesive to the second nonwoven core cover 72, a second rotatable support roll 152 for receiving the second nonwoven core cover 72, a second hopper 154 for holding the SAP 74, a second printing roll 156 for transferring the SAP 74 from the hopper 154 to the second nonwoven core cover 72, and a second hot melt adhesive applicator 158 for applying the hot melt adhesive (or a second portion of hot melt adhesive) 76 to the second nonwoven core cover 72 and the SAP 74 thereon. The second printing unit 134 may also comprise a second pipe 190 in which the additional particle material, such as zeolite particles, may flow through from a reservoir 191 and to the second printing roll 156. A first pipe 192 to add the additional particle material onto the first nonwoven core cover may similarly be added in the first printing unit (see Figure 6). In some embodiments, only the first nonwoven core cover may receive the additional particle material and in other embodiments, only the second nonwoven core cover may receive the additional particle material.

The printing system 130 may also include a guide roller 160 for guiding the formed absorbent core from a nip 162 between the first and second rotatable support rolls 140 and 152.
The optional first hot melt adhesive applicators 136 and 148 and the second hot melt adhesive applicators 146 and 158 may each be configured as a nozzle system which can provide a relatively thin but wide curtain of hot melt adhesive. Both adhesive applicators 136 and 148 may be operated in an intermittent mode, allowing some areas of particle deposition to be on a substrate without adhesive.

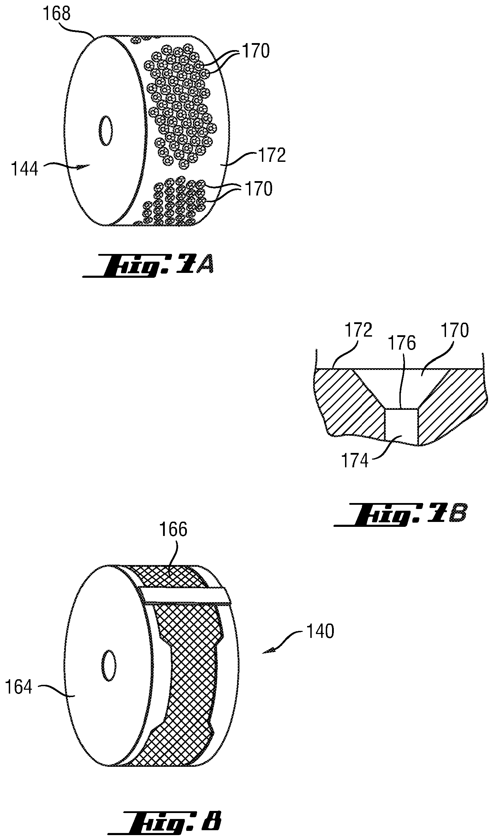

Turning to Fig. 6, portions of the first hopper 142, first support roll 140, first printing roll 144, and first pipe 192 are illustrated. As also shown in Fig. 8, the first rotatable support roll 140, which may have the same structure as the second rotatable support roll 152, comprises a rotatable drum 164 and a vented support in form of a grid 166 for receiving the first nonwoven core cover 64.

As also illustrated in Fig. 7, the first printing roll 144, which has the same structure as the second printing roll 156, comprises a rotatable drum 168 and a plurality of SAP and additional particle material indents 170 in a first surface 172 of the drum 168. The indents 170 also illustrated in Fig. 7 may have a variety of shapes, including cylindrical, conical, or any other shape. The indents 170 may lead to an air passage 174 in the drum 168 and comprise a vented cover 176 for holding SAP 66 and additional particle material 101 in the indent and preventing the SAP 66 and additional particle material 101 from falling or being pulled into the air passage 174.

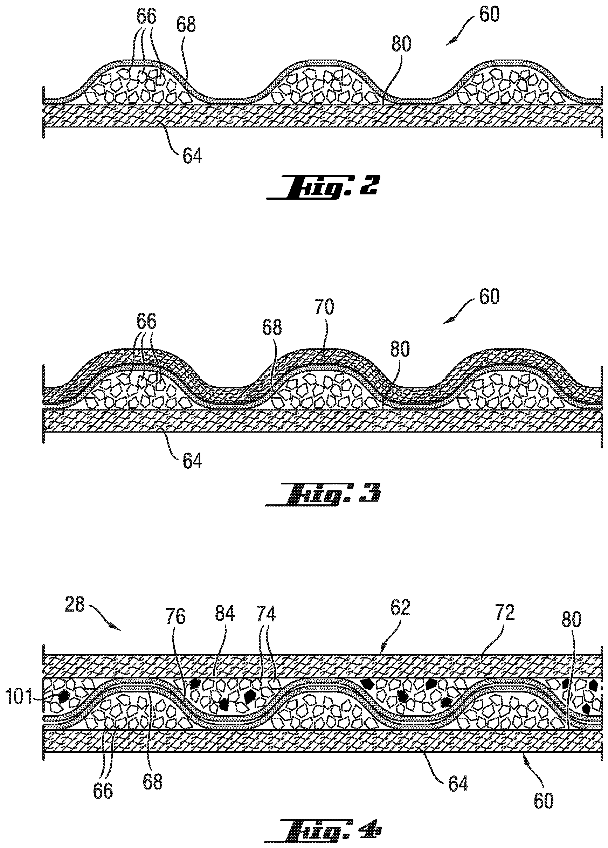

In operation, the printing system 130 receives the first and second nonwoven core covers 64 and 72 into the first and second printing units 132 and 134, respectively, the first nonwoven core cover 64 is drawn by the rotating first support roll 140 past the optional first hot melt adhesive applicator 136 which applies an optional first portion of hot melt adhesive to the first nonwoven core cover 64. A vacuum (not shown) within the first support roll 140 draws the first nonwoven core cover 64 against the support grid 166 and holds the first nonwoven core cover 64 against the first support roll 140. This results in an uneven surface on the first nonwoven core cover 64. The nonwoven core cover 64 will follow the contours of the uneven surface and thereby the nonwoven core cover 64 will assume a ridges and valley shape. The SAP 66 and optionally the additional particle material 101may accumulate in the valleys presented by the first nonwoven core cover 64. The first support roll 140 then carries the first nonwoven core cover 64 past the rotating first printing roll 144 which transfers the SAP 66 and additional particle material 101 from the first hopper 142 to the first nonwoven core cover 64 in a grid pattern. A vacuum (not shown) in the first printing roll 144 may hold the SAP 66 and additional particle material 101 in the indents 170 until the SAP 66 and additional particle material 101 will be delivered to the first nonwoven core cover 64. The vacuum may then be released or air flow through the air passages 174 may be reversed to eject the SAP 66 and additional particle material 101 from the indents and onto the first nonwoven core cover 64. The SAP 66 and additional particle material 101 may accumulate in the valleys presented by the first nonwoven core cover 64. The support roll 140 then carries the printed first nonwoven core cover 64 past the second hot melt adhesive applicator 146 which applies the hot melt adhesive (or a second portion of hot melt adhesive) 68 to cover or to enlace the SAP 66 and additional particle material 101 on the first nonwoven core cover 64. Thereby a first absorbent core precursor 60 is produced. Such an absorbent core precursor is shown in Fig. 2.

The additional particle material 101 is shown in Fig. 2 in one possible configuration. The additional particle material 101, such as zeolite particles, is distributed in a stripe along the longitudinal centerline of the article and of the absorbent core. At least 70%, at least about 80%, or at least about 90% of the additional particle material are within a stripe that is longitudinally centered on the longitudinal centerline and is about 15 mm, or 20 mm, or about 25 mm wide. The additional particle material may be distributed along the entire length of the SAP area, or may be made shorter. The length of the additional particle material deposition may be limited to the length of the SAP as defined by the indentations of the printroll over the width of the longitudinal stripe. A portion (less than about 50%) of the additional particle material may blend with the SAP to provide a uniform, full width blend of SAP and additional particle material.

In some embodiments, only the first nonwoven core cover 64 may have a hot melt adhesive 136, i.e., meaning the second nonwoven core cover 72 may not have the second hot melt adhesive 148. Also, in some embodiments, the additional particle material may be applied only on the second nonwoven core cover. And in some embodiments, specifically the hot melt adhesive is applied only on the first nonwoven core cover and the additional particle material is applied only on the second nonwoven core cover, ensuring that the additional particle material is not between two adhesives that are disposed at and between the first and second nonwoven core covers.

In one embodiment of the process according to the present disclosure the absorbent core precursor 60 will be folded in a C-fold to obtain the absorbent core. Thereby, the first nonwoven core cover envelopes the SAP, additional particle material, and the hot melt adhesive. Alternatively, the SAP, additional particle material, and the hot melt adhesive are applied only to a part of the surface of the nonwoven core cover. The surface extending beyond the area covered by SAP, additional particle material, and hot melt adhesive may then be folded onto the SAP and hot melt adhesive in order to envelope the SAP, additional particle material, and hot melt adhesive, thus forming the absorbent core.

In certain embodiments of the process according to the present disclosure, an additional nonwoven web 70 will be provided and laid down onto the first absorbent core precursor 60 to form an absorbent core. Thereby, the SAP and the hot melt adhesive are sandwiched between the first nonwoven core cover 64 and the additional nonwoven web 70. See for Example Fig. 4. In one embodiment the additional nonwoven web 70 is comprised by the core cover material of the present disclosure as well.

In another embodiment a second absorbent core precursor 62 may be formed simultaneously with the first absorbent core precursor by the following process steps. The second rotatable support roll 152 draws the second nonwoven core cover 72 past the optional first hot melt adhesive applicator 148 which applies an optional first hot melt adhesive to the second nonwoven core cover 72. The second rotatable support roll 152 then carries the second nonwoven core cover 72 past the second printing roll 156 which transfers the SAP 74 and additional particle material 101 from the second hopper 154 to the second nonwoven core cover 72 and deposits the absorbent polymer material 74 in a grid pattern on the second nonwoven core cover 72 in the same manner as described with regard to the first printing unit 132 above. The second hot melt adhesive applicator 158 then applies the hot melt adhesive (or a second portion of hot melt adhesive) 76 to cover or to enlace the SAP 74 and additional particle material 101 on the second nonwoven core cover 72. The first and second nonwoven core covers 64 and 72 then pass through the nip 162 between the first and second support rolls 140 and 152 for compressing the first absorbent core precursor 60 and second absorbent core precursor 62 together to form the absorbent core 28. In some embodiments, the optional first hot melt adhesive, if it is applied at all, may be applied intermittently outside of the area where the additional particle material 101 is placed.

Hence, the uneven surfaces of the vented support grid 166 of the support rolls 140 and 152 respectively determine the distribution of SAP 66 and 74 and additional particle material 101 throughout the absorbent core precursor 60.

### Core cover

The core cover of the present disclosure is a nonwoven fabric made of synthetic fibers.

Synthetic fibers are man-made fibers, comprising fibers derived from natural sources and mineral sources. Example synthetic fibers, which are derived from natural sources include but are not limited to viscose, polysaccharides (such as starch), rayon and lyocell. Example fibers from mineral sources include but are not limited to polyolefin (such as polypropylene or polyethylene) fibers and polyester fibers. Fibers from mineral sources are derived from petroleum.

Nonwoven webs can be formed by direct extrusion processes during which the fibers and webs are formed at about the same point in time, or by preformed fibers which can be laid into webs at a distinctly subsequent point in time. Example direct extrusion processes include but are not limited to: spunbonding, meltblowing, solvent spinning, electrospinning, and combinations thereof. Nonwoven webs often comprise several layers, which may be made from different extrusion processes.

As used herein, the term "spunbonded fibers" refers to small diameter fibers, which are formed by extruding molten thermoplastic material as filaments from a plurality of fine, usually circular capillaries of a spinneret. Spunbond fibers are quenched and generally not tacky when they are deposited onto a collecting surface. Spunbond fibers are generally continuous. The spunbond fibers herein may have diameters of 10µm to 40µm.

As used herein, the term "meltblown fibers" means fibers formed by extruding a molten thermoplastic material through a plurality of fine, usually circular, die capillaries as molten threads or filaments into converging high velocity gas (e.g. air) streams, which attenuate the filaments of molten thermoplastic material to reduce their diameter. Thereafter, the meltblown fibers are carried by the high velocity gas stream and are deposited on a collecting surface to form a web of randomly dispersed meltblown fibers. The meltblown fibers herein may have diameters of 0.2µm to 10µm.

Example "laying" processes include wet-laying and dry-laying. Example dry-laying processes include but are not limited to air-laying, carding, and combinations thereof typically forming layers. Combinations of the above processes yield nonwovens commonly called hybrids or composites.

The term "nonwoven layer" refers to a layer of fibers that has been extruded by the same technique and have been laid down in a single step. Herein "nonwoven layer of meltblown / spunbond fibers" and "meltblown / spunbond layer" are used interchangeably.

The fibers in a nonwoven web are typically joined to one or more adjacent fibers at some of the overlapping junctions. This includes joining fibers within each layer and joining fibers between layers when there is more than one layer. Fibers can be joined by mechanical entanglement, by chemical bonds, thermal bonds, pressure bonds or by combinations thereof.

While spunbond webs provide relatively good resistance to strain, they offer rather poor area coverage, especially in nonwovens having relatively low basis weights, resulting in pores large enough for the SAP or additional particle material to escape. Furthermore, a spunbond nonwoven having a relatively high basis weight, which may provide better area coverage, may not work well as a core cover because of its relatively high stiffness and relatively low water permeability. Additionally it may be more difficult to render a spunbond web with high basis weight hydrophilic In embodiments where the body facing side of the absorbent core is covered by the core cover it is desirable that the core cover is water permeable and hydrophilic. Meltblown layers, due to their smaller average pore size, may be suitable to contain even very small particles, but break or rupture more easily when exposed to strain and offer poor abrasion resistance.

The nonwoven web used for the core cover may comprise three or more nonwoven layers each either consisting of spunbond or meltblown fibers. At least two layers consist of spunbond fibers and one or more of meltblown fibers. The nonwoven layers are arranged in that the one or more meltblown layers are sandwiched between the two or more spunbond layers.

In one embodiment the core cover may comprise three layers, wherein two layers may comprise spunbond fibers (S), one layer may comprise meltblown fibers (M) and wherein the meltblown layer is sandwiched between the spunbond layers, forming a configuration known as SMS. Alternatively, the core cover may comprise four layers, wherein two layers may comprise spunbond fibers, two layers may comprises meltblown fibers and wherein the meltblown layers are sandwiched between the spunbond layers, forming a configuration known as SMMS. In another embodiment the core cover may comprise five or more nonwoven layers, wherein two or more nonwoven layers may comprise spunbond fibers and two, or three, or more nonwoven layers may comprise meltblown fibers and wherein the meltblown layers are sandwiched between the spunbond layers, such as SSMMS, SMMMS, SSMMMS or the like.

It has now been found that by adjusting the ratio of spunbond to meltblown fibers in a nonwoven core cover the retention of SAP can be improved even after the nonwoven has been exposed to strain. Therefore, this ratio may be effectively used to adjust the nonwoven to the requirements of strain intense production processes. In such a nonwoven the spunbond fibers act as an efficient scaffold which is able to stabilize the one or more layers of meltblown fibers. The meltblown fibers on the other side provide a fine net which retains the SAP and additional particle material.

The total basis weight of the nonwoven fabric used for the core cover should be high enough to ensure good area coverage and to provide sufficiently small pores. On the other hand the basis weight should not be too high, so that the nonwoven is still compliant and nonirritating to the skin of the wearer. In preferred embodiments, the total basis weight may range from 8 to 20g/m², or 9 to 16g/m², or 10 to 14g/m², for example 13g/m².

The amount of the spunbond nonwoven fibers in a nonwoven fabric consisting of spunbond and meltblown fibers may be selected such that the content of spunbond fibers ranges from 80 to 95%, or 82 to 90% of the total basis weight of the nonwoven fabric. It has been found that a rather high content of spunbond fibers increases the strain resistance of the nonwoven fabric and helps to reduce the areas in the meltblown layers that are damaged or ruptured when the web is exposed to strain. It has also been found, that in such a core cover even a relatively low amount of meltblown fibers is sufficient for retaining relatively small particles, even after the nonwoven has been strained.

The nonwoven fabric used for the core cover is further characterized in that it does not show large holes after having been exposed to strain, enabling it to effectively retain the SAP during production of the absorbent article and during use. As characterized by air permeability before and after defined straining determined by the method given in the section Test Methods, the nonwoven web of the present disclosure should show an increase in air permeability of less than 18% after having been strained by ε = 10% (wherein ε is prescribed strain, as explained in Test Methods), or less than 20% after having been strained by ε = 15%.

The nonwoven fabric used for core cover of the present disclosure should effectively contain relatively small superabsorbent polymer particles and therefore, it should show an initial air permeability of at most 60 m³/(m²·min), or at most 50 m³/(m²·min), or at most 40 m³/(m²·min)

In certain embodiments the core may be formed by production processes where a vacuum is applied to the nonwoven fabric used for the core cover to hold it on a support and to temporarily immobilize deposited material on the nonwoven. In these embodiments it may be desirable that the nonwoven fabric has an initial air permeability of at least 5 m³/(m²·min), or at least 10 m³/(m²·min), or at least 20 m³/(m²·min).

### Hot melt adhesive

The hot melt adhesive is typically present in a basis weight of 1 - 40 g/m² or 2 - 35 g/m², or 3 - 30 g/m².

Molecular weights herein are given in g/mol unless specified differently.

The hot melt adhesive 68 and 76 serves to cover and at least partially immobilize the SAP 66 and 74 and additional particle material. The hot melt adhesive may at least partially immobilize the SAP and additional particle material by covering or enlacing the SAP and additional particle material. In one embodiment of the present disclosure, the hot melt adhesive 68 and 76 can be disposed essentially uniformly with the SAP 66 and 74 and additional particle material 101. However, in a certain embodiment, the hot melt adhesive 68 and 76 may be provided as a fibrous layer which is at least partially in contact with the SAP 66 and 74 and additional particle material 101 and partially in contact with the nonwoven core cover 64 and 72.

Figs. 2, 3, and 4 show such a structure, and in that structure, the SAP 66 and 74 and additional particle material 101 is provided as a discontinuous layer on a nonwoven core cover 64 and 72. A layer of fibrous hot melt adhesive 68 and 76 is laid down onto the layer of SAP 66 and 74, such that the hot melt adhesive 68 and 76 is in direct contact with the SAP 66 and 74 and additional particle material 101, but also in direct contact with a surface 80 and 84 of the nonwoven core cover 64 and 72, in areas where the nonwoven fabric is not covered by the SAP 66 and 74 and additional particle material 101. This imparts an essentially three-dimensional structure to the fibrous layer of hot melt adhesive 68 and 76. In other words, the hot melt adhesive 68 and 76 undulates between the SAP 68 and 76 and additional particle material 101 and the surface of the nonwoven core cover 64 and 72. As shown in Fig. 4, the additional particle material 101, such as zeolite particles, may be on only one, for example the second nonwoven core cover.

Thereby, the hot melt adhesive 68 and 76 may cover the SAP 66 and 74 and additional particle material 101, and thereby immobilize this material. In a further aspect, the hot melt adhesive 68 and 76 bonds to the nonwoven core cover 64 and 72 and thus affixes the SAP 66 and 74 and additional particle material 101 to the nonwoven core cover 64 and 72. Thus, in accordance with certain embodiments, the hot melt adhesive 68 and 76 immobilizes the SAP 66 and 74 when wet, such that the absorbent core 28 achieves a wet immobilization of more than about 50%, or more than about 60%, 70%, 80% or 90% according to the Wet Immobilization Test described in US Appl. Ser. No. 60/936102.

Of course, while the hot melt adhesives disclosed herein provide a much improved wet immobilization (i.e., immobilization of SAP when the article is wet or at least partially loaded), these hot melt adhesives may also provide a very good immobilization of SAP when the absorbent core 28 is dry. The hot melt adhesive comprises at least one thermoplastic polymer in combination with other thermoplastic diluents such as tackifying resins, plasticizers and additives such as antioxidants.

In certain embodiments, the thermoplastic polymer typically has a weight average molecular weight (Mw) of more than 10,000 and a glass transition temperature (T_{g}) usually below room temperature (25°C), or of less than 22°C, or less than 18°C, or less than 15°C. In certain embodiments T_{g} may be above 0°C > T_{g}. In embodiments where the thermoplastic polymer has more than one T_{g} the values given refer to the lowest glass transition temperature. The thermoplastic polymer may also have a softening point, as determined by the ASTM Method D-36-95 "Ring and Ball", in the range between 50 °C and 300 °C. In some embodiments the Mw of the thermoplastic polymer is less than 10,000,000.

In certain embodiments, typical concentrations of the thermoplastic polymer in a hot melt adhesive are in the range of about 20% to about 40% by weight of the hot melt adhesive.
Exemplary polymers are (styrenic) block copolymers including A-B-A triblock structures, A-B diblock structures and (A-B)n radial block copolymer structures wherein the A blocks are non-elastomeric polymer blocks, typically comprising polystyrene, and the B blocks are unsaturated conjugated diene or (partly) hydrogenated versions of such. The B block is typically isoprene, butadiene, ethylene/butylene (hydrogenated butadiene), ethylene/propylene (hydrogenated isoprene), and mixtures thereof.

In exemplary embodiments, the tackifying resin has typically a Mw below 5,000 and a Tg usually above room temperature (25°C), typical concentrations of the tackifying resin in a hot melt are in the range of about 30% to about 60% by weight of the hot melt adhesive. In certain embodiments the tackifying resin has an Mw of more than 1,000.

The plasticizer has a low Mw of typically less than 1,000 and a Tg below room temperature, with a typical concentration of about 0% to about 15% by weight of the hot melt adhesive. In certain embodiments the plasticizer has an Mw of more than 100.

In certain embodiments, the hot melt adhesive 68 and 76 is present in the form of fibers. In some embodiments, the fibers will have an average thickness of about 1 to about 50 micrometers or about 1 to about 35 micrometers and an average length of about 5 mm to about 50 mm or about 5mm to about 30 mm.

Optionally, a part of the hot melt adhesive, for example an amount of 0 - 10 g/m², may already be deposited on the nonwoven core covers 64 and 72 before application of the SAP 66 and 74 and additional particle material for enhancing adhesion of both the SAP 66 and 74, the additional particle material 101, and the rest of the hot melt adhesive 68 and 76, which is deposited after the SAP and additional particle material have been deposited, to the respective nonwoven core covers 64 and 72.

Said part of the hot melt adhesive may be applied to the nonwoven core covers 64 and 72 by any suitable means, but according to certain embodiments, may be applied in about 0.5 to about 1mm wide slots spaced about 0.5 to about 2 mm apart.

### Additional Particle Material

The additional particle material of the present invention is an odor control composition, such as zeolites. Structurally, zeolites are complex, crystalline inorganic polymers based on an infinitely extending framework of AlO₄ and SiO₄ tetrahedra linked to each other by sharing of oxygen ions. This framework structure contains channels or interconnected voids that are occupied by the cations and water molecules. Suitable zeolites for use herein include zeolite A, zeolite P, zeolite Y, zeolite X, zeolite DAY, zeolite ZSM-5, or mixtures thereof. Most preferred are zeolite A and zeolite Y or mixtures thereof. The zeolite may be hydrophobic. This is typically achieved by increasing the molar ratio of the SiO₂ to AlO₂ content such that the ratio of x to y is at least 1, preferably from 1 to 500, most preferably from 1 to 6.

For the simplest zeolites (such as A, X, Y, and higher alumina containing ZSM-5s), the soluble forms are mixed in a specific ratio with water and acid (as necessary to adjust the pH) and allowed to crystallize at a set temperature for a set time. For more complex zeolites such as low alumina ZSM-5, an organic templating agent is added in this step and allowed to crystallize. After filtering, the zeolites are calcined or dried at high temperatures. This calcination removes bound water and in the case of ZSM-5, burns off the organic templating agent. Typically, the crystals as formed are quite small and it may be necessary to increase the particle size. There are two typical processes for increasing size, agglomeration to produce beads or spheres and extrudation to produce cylindrical pellets. Both these process typically require the use of an additional component such as a binder to aid in the agglomeration or extrusion and additional base. There are several types of additional components with alumina and silica as common examples along with clay.

For absorbent articles, the particle size for direct incorporation of an additional particle material, such as zeolite particles, into the absorbent core of the article may be from about 200 µm to about 800µm. In some embodiments, the zeolite particle size may be from about 250µm, 300µm, 350µm, 400µm, 450µm, or 500µm to about 600µm, 650µm, 700µm, 750µm, or 800µm, in any combination. In order to achieve this dimension, the zeolite crystals (0.2 - 1 µm) may be agglomerated or extruded (often with an additional component or binder such as silica or alumina as described previously).

The present invention may include additional particle material such as zeolites mixed with silicas, or may have silicas alone as an odor control composition. Silica, i.e. silicon dioxide SiO₂, exists in a variety of crystalline forms and amorphous modifications, any of which are suitable for use herein. Silicas tend to have a high surface area, and the silica may be in agglomerated form. The silica may be in a highly purified form such that it contains at least about 90%, about 95%, or even about 99% silicon dioxide. The silica may be silica gel having 100% silica content. Alternatively, the silica may be provided from other sources such as metal silicates including sodium silicate. The silica may be used alone or may be mixed with one or more types of zeolites. The ratio of silica to zeolite may be from about 1: 3, about 1:2, about 1:1, about 2:1, about 3:1, about 4:1, or about 5:1.

In general, the amount of additional particle material, such as zeolites, silicas, or blends of both, may be from about 0.05 g to about 1.2 g per article. In some embodiments, the amount of additional particle material may be from about 0.05 g to about 1.0 g, from about 1.0 g to about 1.2 g, from about 0.15 g to about 1.0 g, from about 0.2 g to about 0.8 g, from about 0.3 g to about 0.7 g, from about 0.4 g to about 1.0 g, from about 0.15 g to about 0.3 g, from about 0.10 g to about 0.3 g, or from about 0.05 g to about 0.3 g.

The ratio of additional particle material to SAP in the cavities of the printroll is less than about 1, in some cases less than about 0.5 or less than about 0.3, or less than about 0.1. The ratio of additional particle material to SAP may be from 0 to 0.1 outside the stripe that contains the majority of the additional particle material, whereas the ratio may be from about 0.1 to about 1 inside of the stripe.

Other particle material that may be incorporated into the absorbent core include other odor control compositions, such as activated carbon or silicas. Additional particle materials may also include porous materials preloaded with functional chemicals such as perfumes and/or other materials.

### Test Methods

Using a TexTest Instruments Air Permeability Tester FX 3300 LABOTESTER III (available from TexTest Instruments, Schwerzenbach, Switzerland) or equivalent, measure the air permeability of the samples according to EDANA 140.2-99 with the following settings.

Samples are conditioned 24 hours and measured at 23°C, 50% relative humidity. Samples that are intended to be strained are conditioned before the strain is applied. The straining has to be carried out at 23°C, 50% relative humidity as well.

Using a circular test area of 20 cm² and a pressure drop of Δp = 125 Pa
Report results in m³/(m²·min) as the arithmetic mean of 5 single measurements taken on different samples.

### Straining method and apparatus

The straining is suitably exercised with an apparatus as described in the following. A suitable device shall have two clamps. The two clamps have a longer edge defining their width. The width of the clamps is 200 mm and the clamps are capable of holding the test piece securely across their full width without damage. The clamps shall be oriented in such that their longer edges are parallel and shall be movable in a direction perpendicular to their longer edges. The device shall be capable of extending a test sample at a constant rate of 3cm/sec to a predetermined length (by moving the two clamps away from each other, see below).

The clamps will be suitable to the task of securely holding the sample without damaging it and have a clampdown force enough to hold the sample securely without slippage in the strained mode, and have a smooth surface from which the areas of the sample in contact with the clamps will not be damaged.

The straining procedure shall consist of the following steps:
Cut a web sample to 50 cm length in the intended direction of straining, and 15 cm in the direction perpendicular to the direction of straining;
Secure the sample between the pair of clamps in such that the sample will be strained in machine direction of the nonwoven sample (machine direction being the direction of production of the nonwoven).

Move the second clamp away from the first clamp carefully just until the sample reaches its original full flat-out length, i.e. it should be wrinkle-free and without bows between the clamps, however the sample will not be strained during this step over its original length. Stop the clamps in the position when this state is reached. Measure and record the unstrained length *l₀* as the edge-to-edge distance between the clamps (all lengths are suitably measured with an accuracy of +/- 1mm). The unstrained length *l₀* should be 30 cm.

Stain the sample at a rate of about 3cm/sec until the strained length l = l₀ + Δl is reached, measured as the edge-to-edge distance between the clamps, where Δl = l₀ · ε / 100 is the elongation and *ε* the prescribed strain (expressed in %). Stop the clamps in this position and hold them for between 1 and 3 seconds. Then move the clamps back to a position where the sample is hanging freely between them and not experiencing any strain, and remove the sample.

The air permeability of the strained samples shall be measured immediately after having strained them following the above procedure. The area of the sample submitted to air permeability testing shall be that which has been in the central position of the straining, i.e. at approximately equal distance between the two clamps in the direction of the straining, and between the free edges in the direction perpendicular to the direction of straining.

Unstrained samples shall be measured as obtained, e.g. from a roll. The samples are to be handled with care and no excessive crumpling or other mechanically stressful treatments should be exercised on them prior to measurement.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A process for forming an absorbent component of an absorbent article, the process comprising:
providing a print roll having a radial outer surface and a rotational axis, wherein a plurality of cavities are defined in the radial outer surface;
rotating the print roll about the rotational axis;
providing a superabsorbent polymer particle material at a second input zone on the print roll;
providing a different particle material at a first input zone on the print roll, wherein the different particulate material is an odor control composition comprising zeolite and/or silicas;
partially filling at least some of the cavities with the different particle material at the first input zone during the rotation of the print roll;
partially filling at least some of the cavities with the superabsorbent polymer particle material at the second input zone during the rotation of the print roll;
holding the superabsorbent polymer particle material and the different particular material in the at least some cavities between the input zones and the depositing zone using a vacuum;
bringing a substrate into a position proximate to or in contact with the print roll in a depositing zone;
depositing the superabsorbent polymer particle material on portions of the substrate in the depositing zone;
depositing the different particle material on portions of the substrate in the depositing zone wherein at least 70% of the different particle material is placed in a stripe substantially along a longitudinal axis of the substrate; and
applying a hotmelt adhesive to the deposited superabsorbent polymer particle material and the deposited different particle material.

2. The process of claim 1, wherein the substrate is a nonwoven.

3. The process of either of claims 1 or 2, wherein the different particle material is deposited before the superabsorbent polymer particle material.

4. The process of any preceding claim, further providing areas on the print roll free of the cavities.

5. The process of claim 4, wherein the superabsorbent polymer particular material or the different particle material in the areas are not deposited in the areas on the print roll free of the cavities.

6. The process of any preceding claim, wherein the different particle material comprises zeolites.

7. The process of claim 1, further providing the step of turning off the vacuum in the depositing zone.

8. The process of either of claims 1 or 7, further providing a positive pressure to the at least some cavities in the depositing zone.

9. The process of any of claims 1, 7 or 8, further providing the step of filling at least 10% of a void volume at the at least some cavities with the different particle material.

10. The process of claim 1, wherein the stripe is 20 mm wide.

11. The process of any of claims 1 or 7-10, including providing the superabsorbent polymer having a weight that is greater than or equal to 80% of a weight of the absorbent component.

12. The process of any of claims 1 or 7-11, including depositing a second substrate onto the superabsorbent polymer material.

## Patentansprüche

1. Verfahren zum Ausbilden einer Absorptionskomponente eines Absorptionsartikels, das Verfahren umfassend:
Bereitstellen einer Druckwalze, die eine radiale Außenoberfläche und eine Drehachse aufweist, wobei eine Vielzahl von Hohlräumen in der radialen Außenoberfläche definiert sind;
Drehen der Druckwalze um die Drehachse herum;
Bereitstellen eines Superabsorberpolymerteilchenmaterials an einem zweiten Eingangsbereich an der Druckwalze;
Bereitstellen eines unterschiedlichen Teilchenmaterials an einem ersten Eingangsbereich an der Druckwalze, wobei das unterschiedliche teilchenförmige Material eine Geruchskontrollzusammensetzung, umfassend Zeolith und/oder Kieselsäuren, ist;
teilweises Füllen wenigstens einiger der Hohlräume mit dem unterschiedlichen Teilchenmaterial an dem ersten Eingangsbereich während der Drehung der Druckwalze;
teilweises Füllen wenigstens einiger der Hohlräume mit dem Superabsorberpolymerteilchenmaterial an dem zweiten Eingangsbereich während der Drehung der Druckwalze;
Halten des Superabsorberpolymerteilchenmaterials und des unterschiedlichen teilchenförmigen Materials in den wenigstens einigen Hohlräumen zwischen den Eingangsbereichen und dem Abscheidungsbereich unter Verwendung eines Vakuums;
Bringen eines Substrats in eine Position nahe gelegen oder in Kontakt mit der Druckwalze in einem Abscheidungsbereich;
Abscheiden des Superabsorberpolymerteilchenmaterials an Abschnitten des Substrats in dem Abscheidungsbereich;
Abscheiden des unterschiedlichen Teilchenmaterials an Abschnitten des Substrats in dem Abscheidungsbereich, wobei wenigstens 70 % des unterschiedlichen Teilchenmaterials in einem Streifen im Wesentlichen entlang einer Längsachse des Substrats platziert werden; und
Aufbringen eines Hotmelt-Klebstoffs auf das abgeschiedene Superabsorberpolymerteilchenmaterial und das abgeschiedene unterschiedliche Teilchenmaterial.

2. Verfahren nach Anspruch 1, wobei das Substrat ein Vlies ist.

3. Verfahren nach einem der Ansprüche 1 oder 2, wobei das unterschiedliche Teilchenmaterial vor dem Superabsorberpolymerteilchenmaterial abgeschieden wird.

4. Verfahren nach einem der vorstehenden Ansprüche, das ferner Flächen an der Druckwalze, die frei von den Hohlräumen sind, bereitstellt.

5. Verfahren nach Anspruch 4, wobei das teilchenförmige Material eines Superabsorberpolymers oder das unterschiedliche Teilchenmaterial in den Flächen nicht in den Flächen an der Druckwalze, die frei von den Hohlräumen sind, abgeschieden wird.

6. Verfahren nach einem der vorstehenden Ansprüche, wobei das unterschiedliche Teilchenmaterial Zeolithe umfasst.

7. Verfahren nach Anspruch 1, das ferner den Schritt eines Abschaltens des Vakuums in dem Abscheidungsbereich bereitstellt.

8. Verfahren nach einem der Ansprüche 1 oder 7, das ferner einen positiven Druck an den wenigstens einigen Hohlräumen in dem Abscheidungsbereich bereitstellt.

9. Verfahren nach einem der Ansprüche 1, 7 oder 8, das ferner den Schritt des Füllens von wenigstens 10 % eines Porenvolumens an den wenigstens einigen Hohlräumen mit dem unterschiedlichen Teilchenmaterial bereitstellt.

10. Verfahren nach Anspruch 1, wobei der Streifen 20 mm breit ist.

11. Verfahren nach einem der Ansprüche 1 oder 7 bis 10, einschließlich des Bereitstellens des Superabsorberpolymers, das ein Gewicht, das größer als oder gleich 80 % eines Gewichts der Absorptionskomponente ist, aufweist.

12. Verfahren nach einem der Ansprüche 1 oder 7 bis 11, einschließlich des Abscheidens eines zweiten Substrats auf das Superabsorberpolymermaterial.

## Revendications

1. Procédé permettant le formage d'un composant absorbant d'un article absorbant, le procédé comprenant :
la fourniture d'un rouleau d'impression ayant une surface externe radiale et un axe de rotation, dans lequel une pluralité de cavités sont définies dans la surface externe radiale ;
la rotation du rouleau d'impression autour de l'axe de rotation ;
la fourniture d'un matériau particulaire polymère superabsorbant au niveau d'une seconde zone d'entrée sur le rouleau d'impression ;
la fourniture d'un matériau particulaire différent au niveau d'une première zone d'entrée sur le rouleau d'impression, dans lequel le matériau particulaire différent est une composition de régulation des odeurs comprenant de la zéolite et/ou des silices ;
le remplissage partiel d'au moins certaines des cavités avec le matériau particulaire différent au niveau de la première zone d'entrée pendant la rotation du rouleau d'impression ;
le remplissage partiel d'au moins certaines des cavités avec le matériau particulaire polymère superabsorbant au niveau de la seconde zone d'entrée pendant la rotation du rouleau d'impression ;
le maintien du matériau particulaire polymère superabsorbant et du matériau particulaire différent dans les au moins certaines cavités entre les zones d'entrée et la zone de dépôt à l'aide d'un vide ;
le fait d'amener un substrat dans une position à proximité du rouleau d'impression ou en contact avec celui-ci dans une zone de dépôt ;
le dépôt du matériau particulaire polymère superabsorbant sur des parties du substrat dans la zone de dépôt ;
le dépôt du matériau particulaire différent sur des parties du substrat dans la zone de dépôt dans lequel au moins 70 % du matériau particulaire différent est placé en une bande sensiblement le long d'un axe longitudinal du substrat ; et
l'application d'un adhésif thermofusible sur le matériau particulaire polymère superabsorbant déposé et le matériau particulaire différent déposé.

2. Procédé selon la revendication 1, dans lequel le substrat est un non-tissé.

3. Procédé selon l'une ou l'autre des revendications 1 ou 2, dans lequel le matériau particulaire différent est déposé avant le matériau particulaire polymère superabsorbant.

4. Procédé selon l'une quelconque revendication précédente, fournissant en outre des aires sur le rouleau d'impression exemptes des cavités.

5. Procédé selon la revendication 4, dans lequel le matériau particulaire polymère superabsorbant ou le matériau particulaire différent dans les aires ne sont pas déposés dans les aires sur le rouleau d'impression exemptes des cavités.

6. Procédé selon l'une quelconque revendication précédente, dans lequel le matériau particulaire différent comprend des zéolites.

7. Procédé selon la revendication 1, fournissant en outre l'étape de désactivation du vide dans la zone de dépôt.

8. Procédé selon l'une ou l'autre des revendications 1 ou 7, fournissant en outre une pression positive aux au moins certaines cavités dans la zone de dépôt.

9. Procédé selon l'une quelconque des revendications 1, 7 ou 8, fournissant en outre l'étape consistant au remplissage d'au moins 10 % d'un volume de vide au niveau des au moins certaines cavités avec le matériau particulaire différent.

10. Procédé selon la revendication 1, dans lequel la bande présente une largeur de 20 mm.

11. Procédé selon l'une quelconque des revendications 1 ou 7 à 10, comportant la fourniture du polymère superabsorbant ayant un poids qui est supérieur ou égal à 80 % d'un poids du composant absorbant.

12. Procédé selon l'une quelconque des revendications 1 ou 7 à 11, comportant le dépôt d'un second substrat sur le matériau polymère superabsorbant.
